(19) Europäisches Patentamt / European Patent Office / Office européen des brevets

(11) **EP 3 060 192 B1**

(12) **EUROPEAN PATENT SPECIFICATION**

(45) Date of publication and mention of the grant of the patent:
**14.10.2020 Bulletin 2020/42**

(51) Int Cl.:
*A61K 8/26* (2006.01)  *A61K 8/27* (2006.01)
*A61Q 11/00* (2006.01)  *A61K 8/19* (2006.01)
*A61K 8/21* (2006.01)

(21) Application number: **14795855.7**

(22) Date of filing: **24.10.2014**

(86) International application number:
**PCT/GB2014/053177**

(87) International publication number:
**WO 2015/059492 (30.04.2015 Gazette 2015/17)**

(54) **DENTAL COMPOSITION**

**DENTALE ZUSAMMENSETZUNG**

**COMPOSITION DENTAIRE**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **24.10.2013 GB 201318813**

(43) Date of publication of application:
**31.08.2016 Bulletin 2016/35**

(73) Proprietor: **Queen Mary University of London London E1 4NS (GB)**

(72) Inventors:
- **HINE, Corwin Ezra**
  **London E1 2AD (GB)**
- **FRANKS, Mark Andrew**
  **London E1 2AD (GB)**
- **PATEL, Mangala**
  **London E1 2AD (GB)**
- **HILL, Robert**
  **London E1 2AD (GB)**
- **PARKER, Sandra**
  **London E1 2AD (GB)**
- **KARPUKHINA, Natalia**
  **London E1 2AD (GB)**

(74) Representative: **Bassil, Nicholas Charles et al Kilburn & Strode LLP Lacon London 84 Theobalds Road London WC1X 8NL (GB)**

(56) References cited:
**EP-A1- 2 039 254          EP-A2- 0 368 420**
**WO-A1-2013/057519      JP-A- S5 538 328**
**JP-A- S5 545 619          JP-A- S5 545 619**
**US-A- 4 296 094          US-A1- 2007 122 358**
**US-A1- 2012 208 895**

- **LOREDANA TAMMARO ET AL: "Effect of layered double hydroxide intercalated with fluoride ions on the physical, biological and release properties of a dental composite resin", JOURNAL OF DENTISTRY, vol. 42, no. 1, 1 January 2014 (2014-01-01), pages 60-67, XP055158816, ISSN: 0300-5712, DOI: 10.1016/j.jdent.2013.10.019**

## Description

Field of the invention

The present invention relates to dental compositions. In particular, the invention relates to dental compositions for fluoride delivery.

Background to the invention

In preventative dentistry, fluoride remains the primary therapeutic agent in the control of post-eruptive dental caries. Common systems of delivery through systemic water fluoridation and topical fluoride applications such as dentifrice and mouth rinses provide a baseline for protection. However, 'at risk' patients benefit from sustained low-levels of fluoride in the oral cavity to maintain the optimal cariostatic effect at the tooth/oral environment interface.

Whilst many restorative materials used in dentistry often release fluoride, there are very few products that have the ability to uptake fluoride thus being recharged. The only notable exception to this are glass ionomer cements that have a capacity to release fluoride to as well as uptake fluoride from the oral environment and re-release it. However, glass ionomer cements have a limited capacity to do this.

The ability to maintain a low constant level of fluoride in the oral cavity is important in caries prevention. Most fluoride-containing products especially gels and varnishes have only a short term beneficial effect. Many dentists use such products with concentrations of fluoride far higher than would be allowed in conventional toothpastes in order to prolong the exposure to fluoride. However, excessive fluoride leads to dental fluorosis, a mottling of the teeth and an unsightly appearance. It may also lead to the formation of undesirable fluorite ($CaF_2$), rather than the formation of fluorapatite. In the case where fluoride is administered to promote re-mineralisation of carious lesions high fluoride concentrations often result in surface re-mineralisation only. There is a wide range of applications in dentistry where a slow release of fluoride and a rechargeable fluoride system would be attractive. The present invention provides such a system.

Layered double hydroxides (LDHs) are a class of synthetic inorganic frameworks that are arranged into positively charged sheets that are counterbalanced through the intercalation of anions within the hydrated interlayers. LDH has been shown to remove excess fluoride from drinking water sources. Like most inorganic crystalline materials that take up fluoride, LDHs have a very strong affinity for fluoride. For example, hydroxyapatite ($Ca_5(PO_4)_3OH$) readily exchanges its hydroxyl ion for a fluoride ion to form fluorapatite ($Ca_5(PO_4)_3F$), but since fluorapatite is thermodynamically more stable and less soluble than hydroxyapatite the fluoride ion is not released readily. JP S5538328 describes a low-cost preventive for dental caries comprising a hydrotalcite-family compound, effective for the disinfection of bacteria in the oral cavity and neutralisation of lactic acid which is harmless when used continuously. US 4296094 describes a dental cleaning composition comprising about 0.01 to about 30% by weight, based on the composition, of a hydrotalcite compound. JP S5545619 describes a preventative for dental caries in the form of a fine powder which contains a hydrotalcite or hydrotalcite derivative as an active constituent and prevents dental caries by adsorbing and removing bacteria in the mouth and neutralising the acid formed by the bacteria. EP 0368420 describes an oral composition comprising a hydrotalcite-like material for the care of the mouth to inhibit oral bacteria and to neutralize acids produced by oral bacteria. EP 2039254 describes a hybrid inducible release vehicle, a method for preparing such a vehicle, and the use thereof. US 2007/122358 describes a dentifrice composition comprising, (a) a hydrophilic clay material, (b) a phytic acid compound, (c) an effective amount of an oral care active, and (d) a polar solvent carrier; wherein the composition is substantially free of abrasive material. WO 2013/057519 describes the use of: i) a composition comprising Portland cement and phyllosilicate; and/or ii) a modified hexacalcium aluminate trisulfate hydrate product formed by the hydration of a composition comprising Portland cement and phyllo - silicate, as an additive in a dental adhesive. Tammaro et al. (2014) (J. Dent. 42. 1. 60-67) describes the effect of layered double hydroxide intercalated with fluoride ions on the physical, biological and release properties of a dental composite resin. US 2012/0208895 describes a composite material for biomedical application, in particular dental applications, which possesses self-healing capacity and is able to incorporate a system for the release of active ingredients at the stage of application and use. The inventors have surprisingly found that LDHs can not only take up fluoride, but can also rerelease it readily.

The present invention discloses dental compositions for use and dental devices incorporating a composition comprising a rechargeable fluoride delivery system wherein the rechargeable fluoride delivery system comprises a layered double hydroxide.

Detailed description of the invention

According to a first aspect the invention relates to a dental composition for use in the treatment or prevention of caries wherein the composition comprises a rechargeable fluoride delivery system wherein the rechargeable fluoride delivery system comprises a layered double hydroxide with the formula $M(II)_{1-x}M(III)_x(OH)_2(A^{n-})_{x/n}yH_2O$, where

M(II) is a divalent cation

M(III) is a trivalent cation

A is a chloride anion

x is a value between 0.2 and 0.33

y is an integer between 1 and 10

n has a value of 1;

wherein the rechargeable fluoride delivery system can be replenished with fluoride.

In an embodiment of the invention, M(II) may be zinc (Zn), magnesium (Mg), calcium (Ca), nickel (Ni) or copper (Cu). As described herein, M(II) is Zn.

In another embodiment of the invention, M(III) may be aluminium (Al), gallium (Ga) or iron (Fe). As described herein, M(III) is Al.

A represents an exchangeable anion with a negative charge. As described herein, A is chlorine (Cl) .

As described herein, M(II) is Zn and M(III) is Al with a Zn:Al ratio between 0.5 and 2.0.

References to atomic elements include the corresponding ion unless the context specifies otherwise, e.g. chlorine/chloride, fluorine/fluoride etc. References to M(II) cations include the designations $Zn^{2+}$, $Mg^{2+}$, $Ca^{2+}$, $Ni^{2+}$, $Cu^{2+}$ for the metal cations defined above. M(II) may be written as $M^{2+}$ also. References to M(III) cations include the designations $Al^{3+}$, $Ga^{3+}$ or $Fe^{3+}$ for the metal cations defined above. M(III) may be written as $M^{3+}$ also. References to the anion A include the designations $Cl^-$ for the anions defined above.

A dental composition as used herein is any composition which can be used in the dental field. The dental composition of the present invention can be used, for example, to prepare a dental filling material, crown and bridge material, veneer material, inlay or onlay, pit and fissure sealant or bonding agent or an adhesive.

As described herein, the dental composition comprises 0.1 to 25 weight percent, or more preferably 5 to 15 weight percent, of a rechargeable fluoride delivery system wherein the rechargeable fluoride delivery system comprises a layered double hydroxide with the formula

$$M(II)_{1-x}M(III)_x(OH)_2(A^{n-})_{x/n}yH_2O.$$

A dental composition of the present invention may also be a toothpaste. As described herein, the toothpaste comprises 0.1 to 10% by weight of a rechargeable fluoride delivery system wherein the rechargeable fluoride delivery system comprises a layered double hydroxide with the formula

$$M(II)_{1-x}M(III)_x(OH)_2(A^{n-})_{x/n}yH_2O.$$

Fluoride varnishes are widely applied to teeth to protect against caries. Generally, these varnishes are light cured resins based on bis-glycidyl dimethacrylate or urethane dimethacrylate containing sodium fluoride. Incorporating a water-soluble fluoride salt into such a resin generally results in rapid water uptake and the resin swelling and cracking to release the fluoride. Consequently these varnishes do not survive for more than a week after application. A varnish based on a layered double hydroxide would last longer as it does not need to crack to release the fluoride.

In an embodiment of the invention, the dental composition is a fluoride varnish.

A dental cement is a mixture of a powdered material and a liquid. A variety of dental cements have been used as restorative filing materials and also adhesives to retain restorations or devices in a fixed position within the oral cavity. Some examples include glass ionomers, resin modified glass ionomers, compomers and composite resins. Dental cements also include luting agents.

In an embodiment of the invention the dental composition is a dental cement. As described herein, the dental cement comprises 0.1 to 25 weight percent of a layered double hydroxide.

A bonding agent is used as an interface to bond the composite to the tooth surface. In an embodiment of the invention the dental composition is a bonding agent.

In an embodiment of the invention the dental composition is a dental resin.

In an embodiment of the invention the dental composition is a composite.

As described herein, the dental composition is a flowable composite. As used herein a "flowable composite" is a dental restorative material that can be dispensed from a syringe or other storage container having a relatively small exit orifice. As described herein, the flowable composite is a restorative that may be applied to a region of a tooth for restoration. Flowable composites were designed to be thixotropic. Thixotropy is a reversible structural breakdown of a material that occurs when the material is stressed. This means that when the material is being syringed, the high stress from syringing breaks down some of the structure (e.g. hydrogen bonding) allowing the material to flow. But when the material is placed into the cavity, it will not flow ('non-drip') because the hydrogen bonding structure quickly recovers.

Dental polymers are commonly used in restorative dentistry. Examples include polyethyl methacrylate, polymethyl methacrylate, hydroxyethyl methacrylate and tetrahydrofurfuryl methacrylate. As described herein, the dental composition is a dental polymer.

Curing refers to the process of hardening a polymer by the application of heat, light, catalyst or curing agents. As described herein, the polymer is room temperature, heat or light curable. A room temperature curable polymer implies that the polymer can be cured at temperatures around 20-30°C, suitably from 20-26°C, for example 23°C, or the standard room temperature of 25°C. A heat curable polymer requires the application of heat in order to cure it. A light curable polymer requires the presence of light for example visible or UV light for curing.

A rechargeable fluoride delivery system as used herein refers to a fluoride delivery system in which the fluoride content when depleted can be replenished. The baseline level of fluoride in saliva is 0.03ppm or less, depending on the fluoride content in drinking water and the use of fluoride containing dental products. These levels are usually not sufficient for individuals at high risk of developing caries. A significant cariostatic effect could be achieved in such individuals if salivary fluoride levels can be sustained at 0.1ppm.

As described herein, the dental composition further comprises other polymers. Examples include but are not limited to polyolefins, polycarbonates, silicones and silicone-like polymers such as styrene-ethylenebutylene-styrene. As described herein, the dental composition comprises silicone. Silicones may be addition silicones or condensation silicones. Non-limiting examples of silicone polymers include polysiloxanes such as vinyl-terminated polydimethylsiloxane, hydroxyl-terminated polydimethylsiloxane and polydimethyl siloxane.

In a second aspect the invention provides a dental device incorporating a composition according to the first aspect.

Dental devices include, but are not limited to, dentures (complete and partial), orthodontic brackets, orthodontic retainers, splints, crowns and bridges. A denture is a removable replacement for missing teeth and surrounding tissue. A denture may be complete or partial. In an embodiment of the invention the dental device is a denture.

In orthodontic treatment metal brackets are bonded to the enamel surface of the teeth for the fixation of metal wires. The metal wires and brackets impede the removal of plaque and consequently about 50% of patients have some caries following treatment. Glass ionomer cements and composite resins are currently used to bond orthodontic brackets to the tooth surface, but as orthodontic treatments may last for six months or more, it is desirable to use a composition that can be recharged with fluoride. The technology could be extended to include coatings placed upon archwires and brackets, or impregnated into elastomeric materials used in orthodontic elastics. In an embodiment of the second aspect, the dental device is an orthodontic bracket.

Orthodontic retainers are often used near the end of the course of orthodontic treatment to hold the teeth in place. Examples of retainers include Hawley, Essix and bonded. In an embodiment of the invention the dental device is an orthodontic retainer.

An occlusal splint is a device primarily used to prevent tooth and joint damage from grinding, clenching and temporomandibular joint dysfunction. In an embodiment of the invention the dental device is an occlusal splint. In another embodiment, the dental device is an orthodontic splint.

A crown is a cap that covers a tooth. It may be made from metal and/or porcelain and is fixed on top of the tooth. In an embodiment of the invention the dental device is a crown. A bridge is a fixed replacement for missing teeth. In an embodiment of the invention the dental device is a bridge.

A mouth guard is a device used to protect teeth from physical injury, for example in a contact sport. A night guard is a device used to ameliorate the negative impacts of bruxism. Both these devices may also be used to provide topical treatment for example by incorporating fluoride within the resin used in their manufacture. As described herein, the dental device is a mouth guard. As described herein, the dental device is a night guard.

It is to be appreciated that the disclosure is not restricted to the devices listed in this application and other devices are also envisaged. For example, a bleaching tray.

As described herein, there is provided a dental composition or device according to the first or second aspects for use in treating or preventing caries. Caries refers to a cavity within the tooth structure that is caused by acid release from a bacterial infection. The term "caries" and "dental caries" are used interchangeably.

Treating caries refers to slowing or stopping the progression of caries. Preventing caries refers to stopping active caries, delaying the onset of caries or reducing the occurrence of new caries in an individual. Caries can be prevented in a number of ways including reducing the number of specific oral bacteria, or changing the types of oral bacteria, or changing the relative number of bacteria.

The dental composition of the invention may also be used as a silicone soft lining material, for example for adaptation to base of denture or denture border adjustment.

As described herein, there is provided a method of treating or preventing caries using a composition of the invention. The method of treatment may be in human or non-human animals (e.g. companion non-human mammals such as cats, or dogs, or agricultural non-human mammal species such as horse, cow, sheep, pig or goat). References to methods of treatment therefore include the use of a composition of the invention in the manufacture of a medicament for the treatment or prevention of caries.

Preferred features of the second aspect of the invention are as for the first aspect *mutatis mutandis.*

The invention will now be described by way of illustration with reference to the following Examples and figures in which:

Fig. 1 shows powder X-ray diffraction patterns for LDH, CSLDH and cellulose

Fig. 2 shows the adsorption isotherms of fluoride on LDH, CSLDH and cellulose at 25 °C

Fig. 3 shows the adsorption isotherm of fluoride on LDH at 25 °C

Fig. 4 shows the $^{19}$F Magic Angle Spinning (MAS) NMR of NaF treated LDH

Fig. 5 shows the release of fluoride upon placing NaF treated polymeric discs in deionised water

Fig. 6 shows the release of fluoride from control and PEM/THFM containing LDH samples (n=5)

Fig. 7 shows the release of fluoride from PMMA samples containing preloaded LDH with fluoride against time (n=4)

Fig. 8 shows the comparison of release of fluoride from LDH-containing PMMA samples and LDH-containing PEM samples after initial charging (n=5)

Fig. 9 shows the cumulative release of fluoride from control PEM discs (time in hours)

Fig. 10 shows the comparison between average amounts of fluoride released in ppm from samples with LDH powder preloaded with fluoride.

The following Examples are intended to illustrate the invention and are not to be construed as limiting the invention:

Examples

Example 1: Synthesis of LDH

Methods described by Mandal and Mayadevi (Chemosphere 72 (2008) 995-998) were followed.

Briefly, 0.852g Zinc Chloride (ZnCl$_2$) and 0.8335 g aluminium chloride (AlCl$_3$) were added to 25 cm$^3$ of deionised H$_2$O to make a 0.5M solution. This ZnCl$_2$-AlCl$_3$ salt solution was titrated simultaneously with 2M NaOH from 2 burettes into a beaker containing 25 cm$^3$ of deionised water. The flow rate of NaOH into the beaker was adjusted to ensure that a pH of 10 $\pm$1 was maintained throughout, and was measured continuously with a pH electrode. The solution was stirred continuously. The final solution was left to age for 24h, after which the solution was topped up to 100cm$^3$ by adding deionised H$_2$O and divided into 4 universal tubes containing 25mls each. These samples were centrifuged for 3 min at 3000 rpm. The supernatant was pipetted off and pH checked with litmus paper, before rinsing the pellet and resuspending it in 25 cm$^3$ deionised H$_2$O and centrifuging again. This was repeated until the supernatant became neutral to litmus. The solids were left in the universal tubes for 36 h at 80 °C to ensure they were completely dehydrated. Samples were removed from the oven and ground to a fine powder.

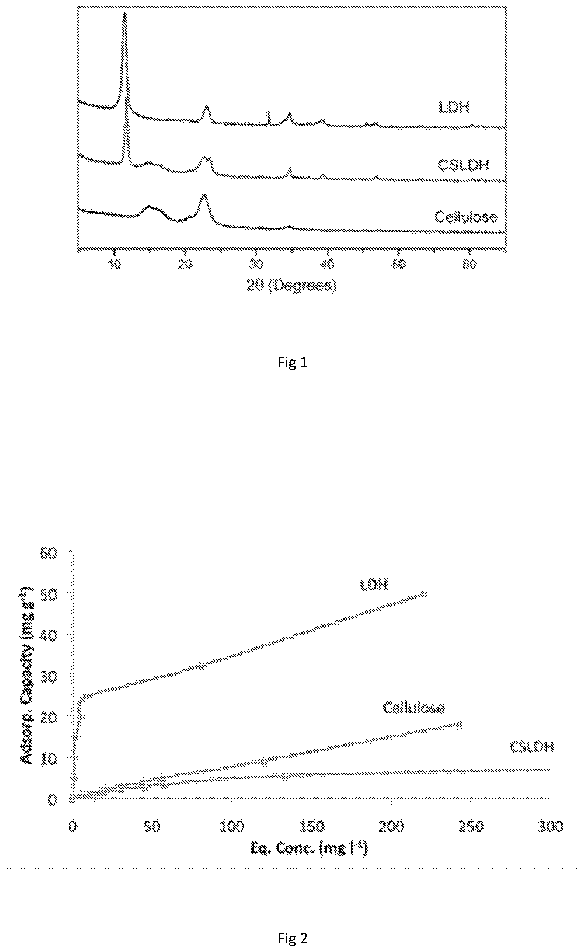

This same procedure was repeated to make a cellulose supported layered double hydroxide (CSLDH) as described by Mandal and Mayadevi. The CSLDH samples were produced by taking 3.371 g cellulose powder which was soaked in 50 cm$^3$ deionised H$_2$O for 30 min at 37 °C, after which the salt solution as described above and NaOH was titrated into the beaker containing the suspended cellulose solution. The solution was stirred continuously. The LDH and CSLDH were characterised using powder X-ray diffraction (XRD). The diffraction pattern relating to these materials are shown in Fig. 1.

Powder XRD pattern serves as a structural fingerprint of a given material. The diffraction pattern of pure cellulose matched previous studies. The large peak at 11.4°, which is absent in cellulose, corresponds to the interlayer spacing of Zn/Al-LDH and corresponds with that of previous studies by Mandal and Mayadevi. However, in contrast the remaining peaks within the diffraction pattern for LDH are of a reduced intensity and significantly broader when compared with CSLDH. This indicates that whilst the LDH prepared here has the same chemical composition the crystallite size is smaller. Such an observation is important as smaller crystallites are likely to contribute to faster fluoride uptake and release.

Example 2: Adsorption isotherm of fluoride on LDH

Serial dilutions of 0.01M, 0.0075M, 0.005M, 0.0025M, 0.001M, 0.0005M and 0.00001M were made from a sodium fluoride (NaF) stock solution (0.1 M). A fluoride ion selective electrode was calibrated using these concentrations starting with the highest concentration and leaving the probe in the solution and recording the mV reading at 3 min intervals until 2 consecutive readings within $\pm$0.3 mV of were achieved. LDH (0.4 mg) was weighed into 15 ml universal tubes and 10 cm$^3$ of NaF solution (0.01, 0.005, 0.0025, 0.0020, 0.0015, 0.0010, 0.0005 M) added and the samples agitated at 25 °C for 60 min.

After 60min the remaining fluoride in solution was measured with the ion selective electrode for 3min and then repeatedly at intervals until the reading remained consistent ($\pm$0.3 mV). This was repeated for each of the three samples measured. The fluoride uptake could be determined from the decrease in fluoride concentration in solution (Fig 2). Table 1 below shows the adsorption values.

Table 1

| Fluoride concentration | | Cellulose | | LDH | | CSLDH | |
|---|---|---|---|---|---|---|---|
| Initial conc. (M) | Initial conc. (mg/l) | Final conc. (mg/l) | Adsorption Capacity (mg/g) | Final conc. (mg/l) | Adsorption Capacity (mg/g) | Final conc. (mg/l) | Adsorption Capacity (mg/g) |
| 0.0005 | 0.05 | 13.81 | 0.734 | 1.308 | 4.922 | 7.276 | 0.995 |
| 0.001 | 0.1 | 20.622 | 2.183 | 1.378 | 10.152 | 18.655 | 1.692 |
| 0.0015 | 0.15 | 31.337 | 3.234 | 1.925 | 15.264 | 29.61 | 2.42 |
| 0.002 | 0.2 | 44.411 | 4.043 | 5.271 | 19.676 | 45.587 | 2.784 |
| 0.0025 | 0.25 | 55.708 | 5.034 | 7.188 | 24.445 | 57.434 | 3.447 |
| 0.005 | 0.5 | 120.495 | 9.141 | 80.922 | 32.254 | 133.783 | 5.523 |
| 0.01 | 1 | 243.07 | 18.07 | 220.645 | 49.809 | 319.88 | 7.252 |

The equilibrium adsorption capacity ($q_e$) of the LDH at different initial fluoride concentrations was calculated using the formula:

$$q_e = (C_0 - C_f) \times V / W \times 100$$

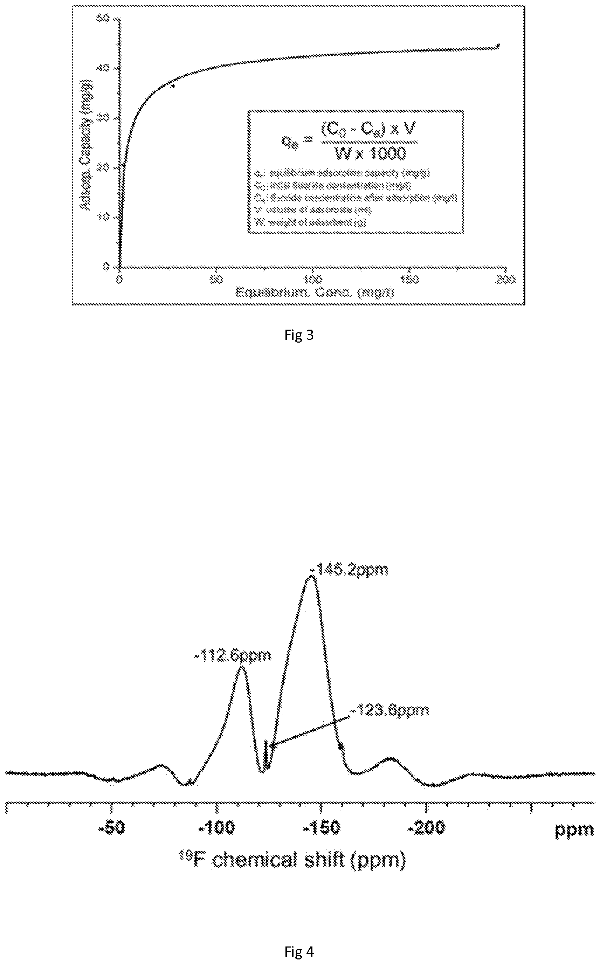

where Co: Initial fluoride conc. (mg l$^{-1}$); $C_f$: Fluoride conc. after adsorption (mg l$^{-1}$); V: volume of NaF sol. (ml); W: weight of adsorbent (g). The LDH was found to exhibit a maximal adsorption capacity of 44.7 mg/g when placed in a 100mM solution of NaF for 1 hour (Fig 3).

The fluoride uptake by the LDH was confirmed by performing $^{19}$F Magic Angle Spinning NMR (Fig 4). The $^{19}$F spectra revealed two broad resonances as -112.6 and -145.2 ppm. This demonstrates the ability of LDH to take up fluoride as no $^{19}$F spectra could be obtained prior to fluoride immersion studies. It also emphasises that fluoride occupies two chemically distinct positions within the layered structure of LDH. The peak at -112.6 ppm probably corresponds to a fluoride bound to zinc, whilst the peak at -145ppm probably corresponds to a fluoride bound to aluminium. The additional sharp peak at -123.6 ppm is associated with fluoride ions that are non-specifically bound adsorbed on the periphery of the material.

Example 3: Desorption of fluoride by LDH

Samples of LDH previously immersed in NaF solutions were centrifuged for 3 min at 3000 rpm, and all supernatant removed. They were then re-suspended in 10ml deionised $H_2O$. Fluoride measurements were made using the fluoride ion selective electrode and readings were taken at 3 and 6min intervals and then samples were left stirring for 1h before re-measuring the fluoride concentration of the solution. Readings were taken again after 72 h, with the samples being stirred continuously. The results are displayed in Table 2 below. Each of the samples measured highlighted that the LDH material slowly releases the adsorbed fluoride over time.

Table 2

| | t = 0 | t = 1h | t = 72h |
|---|---|---|---|
| Adsorption Initial Conc. (M) | Conc. (M) | Conc. (M) | Conc. (M) |
| 0.0005 | 6.66E-07 | 6.84E-07 | 2.19E-06 |
| 0.001 | 1.54E-06 | 1.78E-06 | 4.21 E-06 |
| 0.0015 | 1.94E-06 | 2.28E-06 | 6.17E-06 |

(continued)

| Adsorption Initial Conc. (M) | t = 0 Conc. (M) | t = 1h Conc. (M) | t = 72h Conc. (M) |
|---|---|---|---|
| 0.002 | 3.33E-06 | 4.17E-06 | 8.94E-06 |
| 0.0025 | 6.04E-06 | 6.48E-06 | 1.13E-05 |
| 0.005 | 1.48E-05 | 2.79E-05 | 4.15E-05 |
| 0.01 | 7.26E-06 | 1.05E-05 | 1.45E-05 |

Example 4: Polymeric delivery systems

Polyethyl methacrylate (PEM) and Tetrahydrofurfuryl methacrylate (THFM) discs were made using 5 g of polyethyl methacrylate (PEM) powder and 3ml of Tetrahydrofurfuryl methacrylate (THFM) monomer containing 2.5 wt% Dimethyl para toluidine (DMPT), an amine accelerator. On adding the THFM to the PEM the material formed a gel which polymerised (at room temperature) into a hard, glassy resin. Similarly PEM/THFM & 20% hydroxyethylmethacrylate (HEMA) blanks were made providing a polymeric control with differing hydrophilicity.

The LDH-containing PEM/THFM discs were prepared by adding the required weight % LDH to the PEM powder.

Discs were prepared by using 1mm thick x10 mm diameter moulds and by placing the gel between 2 glass plates (covered with acetate sheets as mould release) and then placed under pressure with a bulldog clip to produce non-porous samples. These were left until set approx. 15min. This procedure was repeated, incorporating 12 wt% LDH with the polymer powder, mixing the two powders until evenly dispersed. Discs removed from the PTFE moulds were trimmed, edges smoothed with silicon carbide paper and weighed.

Analogous systems were created in cold cure polymethyl methacrylate (PMMA). Briefly, self-cured (room temperature cured) PMMA polymeric discs were prepared (addition polymerisation reaction) by mixing the polymer powder (PMMA) with the methyl methacrylate monomer liquid (MMA). The powder was kept at room temperature and the liquid monomer was stored in a dark bottle in the refrigerator at 4 °C approximately. PMMA control discs were prepared by adding 5 grams PMMA powder to 2.3 ml MMA liquid. The powder and liquid were mixed and poured in the PTFE mould of the same dimensions as the one used for PEM/THFM.

Five samples of each polymer PEM/THFM, PEM/THFM/LDH, PEM/THFM/HEMA and PEM/THFM/HEMA/LDH as well as PMMA/THFM and PMMA/THFM/LDH were placed in 15 ml universal tubes with 10 ml 0.01 M NaF solution. These were left agitating for 24 h, after which the fluoride concentration in solution was measured and the adsorption capacity calculated as described previously. The polymeric discs were blot dried and weighed to determine the total absorption of $H_2O$ and NaF. When compared with GIC, the polymeric discs showed faster adsorption of fluoride (data not shown).

Example 5: Fluoride release from polymeric systems in de-ionised water

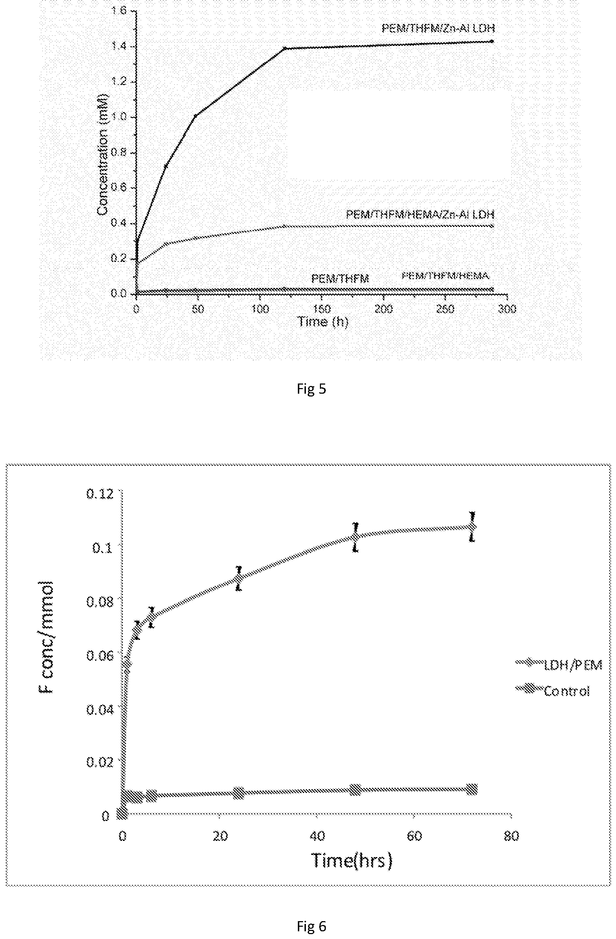

The polymeric discs of each polymer were placed in 5ml de-ionised $H_2O$ and the fluoride concentration measured with a fluoride electrode as a function of time over a 10 day period. The polymers containing no LDH (control: PEM/THFM and PEM/THFM/HEMA) gave no fluoride release. In contrast the polymer samples loaded with LDH resulted in significant fluoride release (Fig 5). The PEM/THFM/LDH polymer gave up to 0.11 mM (2.4 ppm) of fluoride over a 3 day period. (Fig. 6).

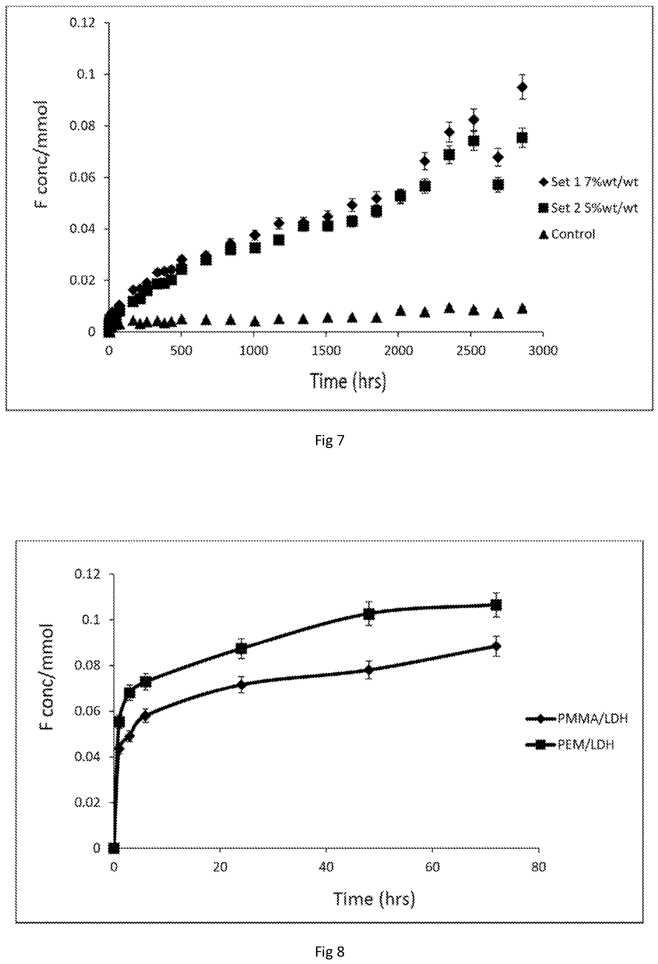

Analogous discs to the ones described above were generated in PMMA containing 5% and 7% wt/wt LDH that had been pre-treated with NaF. These pre-loaded discs were then analysed with respect to their capacity to release fluoride in to a static solution of deionised water. The release was monitored for 120 days identifying a slow-prolonged release of fluoride over this time period (Fig 7).

Example 6: Fluoride release from polymeric systems in artificial saliva

Artificial saliva was prepared as follows: 800 ml deionized water were added in a beaker with a magnetic stirrer and 15.09 grams of tris powder were added slowly until completely dissolved. 44.2 ml HCl was then added and the mix was poured in a plastic bottle and left in an incubator at 37 °C overnight. The next day the pH of the solution was adjusted to approximately 7.25-7.4 by addition of HCl and the bottle was topped up with deionized water to a volume of two litres.

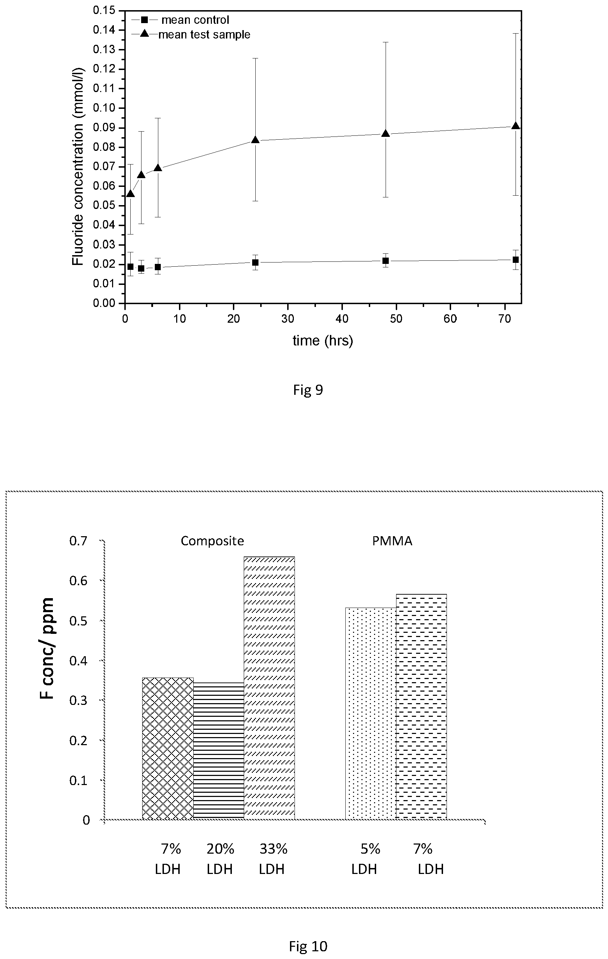

PEM/THFM/LDH and PMMA/THFM/LDH discs analogous to those discussed earlier were investigated to evaluate their capacity to release fluoride within an artificial saliva medium. The release of fluoride, over the same conditions as described previously, was analogous to that produced within aqueous medium (Fig 8). Figure 9 shows release of fluoride from PEM/THFM/LDH discs when compared to control discs. Average amounts of fluoride released from the LDH containing PMMA and PEM/THFM systems studied are shown in Figure 10.

**Claims**

1. A dental composition for use in the treatment or prevention of caries wherein the composition comprises a rechargeable fluoride delivery system wherein the rechargeable fluoride delivery system comprises a layered double hydroxide with the formula

$$M(II)_{1-x}M(III)_x(OH)_2(A^{n-})_{x/n}yH_2O$$

where,

   M(II) is a divalent cation
   M(III) is a trivalent cation
   A is a chloride anion
   x is a value between 0.2 and 0.33
   y is an integer between 1 and 10
   n has a value of 1;

   wherein the rechargeable fluoride delivery system can be replenished with fluoride.

2. The dental composition for use according to claim 1 wherein M(II) is selected from the group consisting of Zn, Mg, Ca, Ni and Cu.

3. The dental composition for use according to claim 1 or 2 wherein M(III) is selected from the group consisting of Al, Ga and Fe.

4. The dental composition for use according to any one of claims 1 to 3, comprising 0.1 to 25 weight percent of the rechargeable fluoride delivery system.

5. The dental composition for use according to any one of claims 1 to 4 wherein the composition is a dental filling material, crown and bridge material, veneer material, an inlay or an onlay, a pit and fissure sealant or a bonding agent or an adhesive.

6. The dental composition for use according to any one of claims 1 to 4 wherein the composition is a toothpaste.

7. The dental composition for use according to any one of claims 1 to 4 wherein the composition is a fluoride varnish, a dental cement, a dental resin or a composite.

8. A dental device incorporating a composition comprising a rechargeable fluoride delivery system wherein the rechargeable fluoride delivery system comprises a layered double hydroxide with the formula

$$M(II)_{1-x}M(III)_x(OH)_2(A^{n-})_{x/n}yH_2O.$$

where,

   M(II) is a divalent cation
   M(III) is a trivalent cation
   A is a chloride anion
   x is a value between 0.2 and 0.33
   y is an integer between 1 and 10
   n has a value of 1;

   wherein the rechargeable fluoride delivery system can be replenished with fluoride.

9. The dental device according to claim 8 wherein M(II) is selected from the group consisting of Zn, Mg, Ca, Ni and Cu.

10. The dental device according to claim 8 or 9 wherein M(III) is selected from the group consisting of Al, Ga and Fe.

11. The dental device according to any one of claims 8 to 10, wherein the composition comprises 0.1 to 25 weight percent of the rechargeable fluoride delivery system.

12. The dental device according to any one of claims 8 to 11 wherein the device is a denture, orthodontic bracket, orthodontic retainer, splint, crown or bridge.

**Patentansprüche**

1. Dentalzusammensetzung zur Verwendung bei der Behandlung oder Vorbeugung von Karies, wobei die Zusammensetzung ein wiederaufladbares Fluoridabgabesystem umfasst, wobei das wiederaufladbare Fluoridabgabesystem ein Schichtdoppelhydroxid mit der Formel

$$M(II)_{1-x}M(III)_x(OH)_2(A^{n-})_{x/n}yH_2O$$

umfasst, wobei

M(II) ein zweiwertiges Kation ist,
M(III) ein dreiwertiges Kation ist,
A ein Chlorid-Anion ist,
x ein Wert zwischen 0,2 und 0,33 ist,
y eine ganze Zahl zwischen 1 und 10 ist,
n einen Wert von 1 aufweist;
wobei das wiederaufladbare Fluoridabgabesystem mit Fluorid aufgefüllt werden kann.

2. Dentalzusammensetzung zur Verwendung gemäß Anspruch 1, wobei M(II) ausgewählt ist aus der Gruppe bestehend aus Zn, Mg, Ca, Ni und Cu.

3. Dentalzusammensetzung zur Verwendung gemäß Anspruch 1 oder 2, wobei M(III) ausgewählt ist aus der Gruppe bestehend aus Al, Ga und Fe.

4. Dentalzusammensetzung zur Verwendung gemäß einem der Ansprüche 1 bis 3, umfassend 0,1 bis 25 Gewichtsprozent an dem wiederaufladbaren Fluoridabgabesystem.

5. Dentalzusammensetzung zur Verwendung gemäß einem der Ansprüche 1 bis 4, wobei die Zusammensetzung ein Zahnfüllmaterial, Kronen- und Brückenmaterial, Veneermaterial, ein Inlay oder ein Onlay, ein Grübchen- und Fissurenversiegelungsmaterial oder ein Bindemittel oder ein Klebstoff ist.

6. Dentalzusammensetzung zur Verwendung gemäß einem der Ansprüche 1 bis 4, wobei die Zusammensetzung eine Zahnpasta ist.

7. Dentalzusammensetzung zur Verwendung gemäß einem der Ansprüche 1 bis 4, wobei die Zusammensetzung ein Fluoridlack, ein Zahnzement, ein Zahnharz oder ein Verbundstoff ist.

8. Dentalvorrichtung, umfassend eine Zusammensetzung, die ein wiederaufladbares Fluoridabgabesystem umfasst, wobei das wiederaufladbare Fluoridabgabesystem ein Schichtdoppelhydroxid mit der Formel

$$M(II)_{1-x}M(III)_x(OH)_2(A^{n-})_{x/n}yH_2O$$

umfasst, wobei

M(II) ein zweiwertiges Kation ist,
M(III) ein dreiwertiges Kation ist,
A ein Chlorid-Anion ist,
x ein Wert zwischen 0,2 und 0,33 ist,
y eine ganze Zahl zwischen 1 und 10 ist,

n einen Wert von 1 aufweist;
wobei das wiederaufladbare Fluoridabgabesystem mit Fluorid aufgefüllt werden kann.

9. Dentalvorrichtung gemäß Anspruch 8, wobei M(II) ausgewählt ist aus der Gruppe bestehend aus Zn, Mg, Ca, Ni und Cu.

10. Dentalvorrichtung gemäß Anspruch 8 oder 9, wobei M(III) ausgewählt ist aus der Gruppe bestehend aus Al, Ga und Fe.

11. Dentalvorrichtung gemäß einem der Ansprüche 8 bis 10, wobei die Zusammensetzung 0,1 bis 25 Gewichtsprozent an dem wiederaufladbare Fluoridabgabesystem umfasst.

12. Dentalvorrichtung gemäß einem der Ansprüche 8 bis 11, wobei die Vorrichtung eine Zahnprothese, eine orthodontische Klammer, eine orthodontische Haltevorrichtung, ein Splint, eine Krone oder eine Brücke ist.

**Revendications**

1. Composition dentaire pour une utilisation dans le traitement ou la prévention de caries, la composition comprenant un système rechargeable d'apport de fluorure, le système rechargeable d'apport de fluorure comprenant un hydroxyde double lamellaire doté de la formule

$$M(II)_{1-x}M(III)_x(OH)_2(A^{n-})_{x/n}yH_2O$$

dans laquelle,

M(II) est un cation divalent
M(III) est un cation trivalent
A est un anion chlorure
x est une valeur comprise entre 0,2 et 0,33
y est un entier compris entre 1 et 10
n possède une valeur de 1 ;
le système rechargeable d'apport de fluorure pouvant être réalimenté en fluorure.

2. Composition dentaire pour une utilisation selon la revendication 1, M(II) étant choisi dans le groupe constitué par Zn, Mg, Ca, Ni et Cu.

3. Composition dentaire pour une utilisation selon la revendication 1 ou 2, M(III) étant choisi dans le groupe constitué par Al, Ga et Fe.

4. Composition dentaire pour une utilisation selon l'une quelconque des revendications 1 à 3, comprenant 0, 1 à 25 pour cent en poids du système rechargeable d'apport de fluorure.

5. Composition dentaire pour une utilisation selon l'une quelconque des revendications 1 à 4, la composition étant un matériau d'obturation dentaire, un matériau de couronne et de bridge, un matériau de placage, un inlay ou un onlay, un agent de scellement pour puits et fissures ou un agent de liaison ou un adhésif.

6. Composition dentaire pour une utilisation selon l'une quelconque des revendications 1 à 4, la composition étant une pâte dentifrice.

7. Composition dentaire pour une utilisation selon l'une quelconque des revendications 1 à 4, la composition étant un vernis au fluorure, un ciment dentaire, une résine dentaire ou un composite.

8. Dispositif dentaire incorporant une composition comprenant un système rechargeable d'apport de fluorure, le système rechargeable d'apport de fluorure comprenant un hydroxyde double lamellaire doté de la formule

$$M(II)_{1-x}M(III)_x(OH)_2(A^{n-})_{x/n}yH_2O$$

dans laquelle,

M(II) est un cation divalent
M(III) est un cation trivalent
A est un anion chlorure
x est une valeur comprise entre 0,2 et 0,33
y est un entier compris entre 1 et 10
n possède une valeur de 1 ;
le système rechargeable d'apport de fluorure pouvant être réalimenté en fluorure.

9. Dispositif dentaire selon la revendication 8, M(II) étant choisi dans le groupe constitué par Zn, Mg, Ca, Ni et Cu.

10. Dispositif dentaire selon la revendication 8 ou 9, M(III) étant choisi dans le groupe constitué par Al, Ga et Fe.

11. Dispositif dentaire selon l'une quelconque des revendications 8 à 10, la composition comprenant 0,1 à 25 pour cent en poids du système rechargeable d'apport de fluorure.

12. Dispositif dentaire selon l'une quelconque des revendications 8 à 11, le dispositif étant un dentier, un boîtier orthodontique, un appareil de contention orthodontique, une attelle, une couronne ou un bridge.

Fig 1

Fig 2

Fig 3

Fig 4

Fig 5

Fig 6

Fig 7

Fig 8

Fig 9

Fig 10

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP S5538328 B
- US 4296094 A
- JP S5545619 B
- EP 0368420 A
- EP 2039254 A
- US 2007122358 A
- WO 2013057519 A
- US 20120208895 A

**Non-patent literature cited in the description**

- **TAMMARO et al.** *J. Dent.,* 2014, vol. 42 (1), 60-67
- **MANDAL ; MAYADEVI.** *Chemosphere,* 2008, vol. 72, 995-998